# EUROPEAN PATENT APPLICATION

(11) **EP 1 897 530 A1**
(43) Date of publication of application: **12.03.2008**
(21) Application number: 06026413.2
(22) Date of filing: 20.12.2006
(51) Int. Cl.: A61K 8/34, A61K 8/36, A61K 8/37, A61K 8/49, A61K 8/31, A61Q 19/08

(54) **Skin care composition**

(30) Priority: 08.09.2006 EP 06018843
(71) Applicant: DSM IP Assets B.V., 6411 TE Heerlen (NL)
(72) Inventor: The designation of the inventor has not yet been filed
(74) Representative: Rabanus, Birgit

(57) **Abstract**

The present invention relates to an oral composition containing an optimal combination of nutritional ingredients at high concentrations and at optimal ratios in order to maintain and/or achieve health and/or beautiful look of the skin.

## Description

The present invention relates to an oral composition containing an optimal combination of nutritional ingredients at high concentrations and at optimal ratios in order to maintain and/or achieve health and/or a beautiful appearance of the human skin.

The skin is the largest organ of the human body. It is made up of multiple layers of epithelial tissues that guard underlying muscles and organs. As the interface with the surroundings, it plays the most important role in protecting the body against pathogens. It furthermore provides sensory perception, moisture control, shape, regulation of metabolic processes, immune response and insulation against heat and cold. The skin also has the remarkable role of defining our individuality by transmitting information back to the environment about a person's appearance, behavior and health.

The outermost layer of the skin is called epidermis. It forms the waterproof, protective wrap over the body's surface and is made up of stratified squamous epithelium with an underlying basal lamina. It contains no blood vessels, and is nourished by diffusion from the dermis. The main types of cells which make up the epidermis are keratinocytes, with melanocytes and Langerhans cells also present. Epidermis is divided into several layers where cells are formed through mitosis at the innermost layers. They move up the strata changing shape and composition as they differentiate and become filled with keratin. They eventually reach the top layer called stratum corneum and become sloughed off, or desquamated. This process is called keratinization and takes place within weeks. The outermost layer of Epidermis consists of 25 to 30 layers of dead cells. Every day thousands of new skin cells are generated.

The body naturally looses water by constant gentle evaporation through the skin (transepidermal water loss, TEWL). Preventing excessive water loss is exceptionally important in itself - both to the skin itself and to the body as a whole. In the normal epidermis the water content gets less the closer we get to the surface. Water makes up to 70 to 75 % of the weight of the basal layer, but only 10 to 15 wt.-% of the stratum corneum. The presence of an adequate amount of water in the epidermis is important for the general appearance of a soft, smooth and attractive skin.

The stratum corneum is hydrated by the water trapped in lower layers as well as by normal perspiration. If it is deficient in natural moisturising factors or subjected to extreme weather conditions, the skin loses moisture. It becomes dry and taut. As it becomes less supple, its protective function is reduced. The skin chaps, transepidermal water loss increases, and its barrier function is impaired. As the skin ages, sebum (or oil) production slows down. Production of moisture-binding substances also decreases. The skin becomes drier and less able to retain moisture. It assumes a flat, dull appearance and is less smooth. Fine lines and wrinkles begin to form and the skin is less firm.

There is a multitude of different intrinsic and external factors that influence skin condition and foster skin aging. Intrinsic factors are genetic predisposition, the normal aging process, or hormonal status. UV radiation, air pollution, smoking and/or allergenic compounds are external factors which can account for premature skin aging.
As the skin ages chronically it particularly loses elasticity. Chronic exposure to UV radiation (UVB and UVA) leads to premature skin aging through epidermal and dermal damage by oxidative stress and sunburn. Sunburn is the inflammatory reaction of the skin in response to excessive exposure to natural or artificial solar light of the UVB wavelength.
Hyperkeratosis, keratinocyte dysplasia and/or dermal elastosis occur in affected skin areas, clinically presenting as photoaged skin with actinic or solar keratosis. These precancerous lesions show an increased risk for the development of squamous cell carcinoma (SCC). The clinical condition of premature skin aging is accompanied with hyperpigmentation and dilated and twisted microvasculature, i.e. teleangiectasia.

In addition to other influencing factors also nutrition has a strong impact on the skin's condition. Optimization of the diet improves general skin condition and hydration of the skin. A deficiency in essential vitamins and/or fatty acids e.g. has clear cutaneous effects. Therefore for full skin protection, a well-balanced mix of different nutritional substances is essential. Optimally, micronutrients, i.e. vitamins, certain polyunsaturated fatty acids (PUFAs) and other nutritional active compounds such as antioxidants are supported internally, i.e. by oral administration, absorption, and transport to the skin via the blood stream.

PUFAs are known to be important for the preservation of the skin-barrier function and the water content of the skin. They are incorporated into the cell membranes of skin keratinocytes and fibroblasts. Due to their structure, PUFAs enhance membrane fluidity, and thus contribute to more flexible and mobile cell membranes than saturated fatty acids. Oral intake of certain PUFAs such as gamma-linolenic acid or linoleic acid leads to a more moistened and smoother skin.

The present invention relates to an oral composition providing an optimal combination of nutritional ingredients at high concentrations and at optimum ratios, preferably in capsules whereas the composition should show excellent bioavailability.

The present invention relates to a composition for oral intake comprising
i) at least one component selected from the group consisting of polyunsaturated fatty acids, esters of polyunsaturated fatty acids - e.g. ethyl esters, mono-, di- and triglyceridesters - and fish oil; and
ii) at least one component selected from the group consisting of carotenoids, water-soluble vitamins, fat-soluble vitamins, ubichinones and polyphenols,
wherein the amount of the component(s) ii) is in the range of 25 to 80 % by weight, based on the total weight of the composition.

According to the present invention the amount of the component(s) ii) is preferably in the range of 30 to 75 % by weight, more preferably in the range of 40 to 60 % by weight, based on the total weight of the composition.

Polyunsaturated fatty acids, which are suitable according to the present invention, are mono- or polyunsaturated carboxylic acids having preferably 16 to 24 carbon atoms and, in particular, 1 to 6 double bonds, particularly preferably having 4 or 5 or 6 double bonds.

The unsaturated fatty acids can belong both to the n-6 series and to the n-3 series. Polyunsaturated fatty acids of the n-3 series are preferred. Preferred examples of n-3 polyunsaturated acids are eicosapenta-5,8,11,14,17-enoic acid (EPA) and docosahexa-4,7,10,13,16,19-enoic acid (DHA), as well as arachidonic acid (ARA).

Preferred derivatives of the polyunsaturated fatty acids are their esters, for example glycerides and, in particular, triglycerides; particularly preferably the ethyl esters. Triglycerides of n-3 polyunsaturated fatty acids are especially preferred.

The triglycerides can contain 3 uniform unsaturated fatty acids or 2 or 3 different unsaturated fatty acids. They may also partly contain saturated fatty acids.

When the derivatives are triglycerides, normally three different n-3 polyunsaturated fatty acids are esterified with glycerin. In one preferred embodiment of the present invention triglycerides are used, whereby 30 % of the fatty acid part are n-3 fatty acids and of these 25 % are long-chain polyunsaturated fatty acids. In a further preferred embodiment commercially available ROPUFA® '30' n-3 Food Oil (DSM Nutritional Products Ltd, Kaiseraugst, Switzerland) is used.
In another preferred embodiment of the present invention the PUFA ester is ROPUFA^{®} '75' n-3 EE. ROPUFA '75' n-3 EE is refined marine oil in form of an ethyl ester with minimum content of 72 % n-3 fatty acid ethyl ester. It is stabilized with mixed tocopherols, ascorbyl palmitate, citric acid and contains rosemary extract.

According to the present invention it can be advantageous to use naturally occurring oils (one ore more components) containing triglycerides of polyunsaturated fatty acids, for example plant oils.

Preferred oils which comprise triglycerides of polyunsaturated fatty acids are olive oil, sunflower seed oil, evening primrose seed oil, borage oil, grape seed oil, soybean oil, groundnut oil, wheat germ oil, pumpkin seed oil, walnut oil, sesame seed oil, rapeseed oil (canola), blackcurrant seed oil, kiwifruit seed oil, oil from specific fungi and fish oils.

Alternatively other polyunsaturated fatty acids (e. g. omega-3 fatty acids; omega-6 fatty acids) and/or their derivatives may be used.

In a preferred embodiment of the present invention the components ii) (one or more components) are selected from one or more of the following groups:
■ carotenoids (comprising the groups of xanthophylls and carotenes):
   beta-carotene, lycopene, lutein, zeaxanthin, and their esters.
■ water soluble vitamins:
   vitamin C, B₆, B₂, B₁₂, biotin, calcium-pantothenate and pharmaceutically acceptable salts thereof;
**■** fat (lipid) soluble vitamins:
   vitamin A, vitamin E and pharmaceutically acceptable salts and derivatives thereof;
■ ubichinones:
   coenzyme Q 10, vitamin K and pharmaceutically acceptable derivatives thereof;
**■** polyphenols:
   (-)-Epigallocatechin gallate (EGCG), hydroxytyrosol, olive extract, resveratrol, genistein and pharmaceutically acceptable derivatives thereof.

Preferably, the invention relates to a composition for consumption by humans comprising
i) at least one component selected from the group consisting of polyunsaturated fatty acids, esters of polyunsaturated fatty acids such as ethyl esters, mono-, di- and triglyceridesters, and fish oil, and
ii) at least one polyphenol selected from the group consisting of (-)-epigallocatechin gallate, genistein, resveratrol and pharmaceutically acceptable derivatives thereof; and
iii) optionally hydroxytyrosol, pharmaceutically acceptable derivatives thereof and/or olive extract;
wherein the amount of the component(s) ii) and iii) is in the range of 25 to 80 % by weight, based on the total weight of the composition.

In an even more preferred embodiment of the present invention this composition comprises in addition to PUFAs and/or their esters and polyphenol(s) an additional amount of lipid soluble and/or water soluble vitamins, co-enzymes, anti-oxidants, carotenoids and/or their esters.

The composition according to the present invention is most suitable to support healthy skin appearance and beauty.
In particular it
■ supports skin nourishment from inside the body via the blood stream (systemically);
■ fosters hydration and moisturizes the skin;
■ supports skin barrier function (especially if the components ii) (one or more) are selected from the group consisting of biotin, vitamin E and genistein);
**■** provides protection against oxidative stress
   (especially if the components ii) (one or more) are selected from the group consisting of beta-carotene, lycopene, vitamin C, vitamin E, EGCG, hydroxytyrosol and coenzyme Q 10);
**■** provides protection against UV-radiation
   (especially if the components ii) (one or more) are selected from the group consisting of carotenoids - preferred beta-carotene - vitamin E, EGCG and genistein); and
■ has a general anti-aging effect
   (especially if the components ii) (one or more) are selected from the group consisting of carotenoids, vitamin C and vitamin E, EGCG, resveratrol).
   Thus, the composition according to the present invention supports beauty from inside. The composition also promotes skin repair and regeneration upon healing of injuries as well as the physiological renewal process.

Overall the composition is promoting an optimal health, a natural radiance and glow and/or a beautiful look of the skin. According to the present invention the term "beautiful look of the skin" comprises a good blood supply generating a natural pink tone, a natural radiance and glow, a pure, clear appearance lacking major impurities and normal sebum secretion, good elasticity, hydration and moisture of the skin and an intact skin barrier function.

Optimal skin moisture and hydration means little transepidermal water loss and intact skin barrier function. Well-hydrated skin is more firm and toned. Hydration minimizes the appearance of fine lines and wrinkles.
The hydration level of the outermost skin layer (10 - 20 µm depth), the stratum corneum, may be measured with a corneometer. The corneometric measurement allows an interpretation about the condition of the skin, the skin type and the effects of pharmaceutical and cosmetic products.
The principle of the corneometer is based on a capacitance measurement of a dielectric medium. Water has a relatively high dielectric constant (D = 80) in contrast to most other substances (D < 7). Therefore even slightest changes in the hydratation of the skin's surface alter the dielectric constant and accordingly the capacitance of a precision measuring capacitor. A spring in the probe head ensures constant pressure of 3,5 N on the skin. The measuring surface is 49 mm². The reproducibilty and accuracy (± 3%) of the measurement is high and the measurement time is 1 s only (prevents occlusion effects).
Boosting of moisture-binding capability of skin enables it to retain its healthy glow. Super hydrated skin is softer to the touch and has a smoother appearance.

In the event of an injury that damages the skin's protective barrier, the body triggers a response called inflammation, which sends fluids carrying phagocytic white blood cells to the injury site. Once the invading microorganisms have been brought under control, the skin proceeds to heal itself. The ability of the skin to heal even after considerable damage has occurred is due to the presence of stem cells in the dermis and cells in the stratum basale of the epidermis, all of which can generate new tissue.
In healthy skin, the immune defense is intact, inflammation within physiological range and the self healing process functional. The composition as defined above can support skin repair e.g. by occlusion, by partly replacing the lipids or by triggering cellular lipid production.
Epidermal keratinocytes are produced constantly from stem cells in the basal layer of the epidermis. During their migration upwards, the keratinocytes run through a differentiation process. The result of epidermal differentiation is the formation of the stratum corneum, which consists of terminally differentiated, cornified keratinocytes. The process from proliferation to desquamation takes about one month. The composition as defined above supports this renewal process.

### Lycopene and derivatives thereof

The term "lycopene" as used herein includes all-E and Z-stereoisomers. Alternatively a tomato extract which contains high amounts of lycopene can also be used.

### Lutein and derivatives thereof

The term "lutein" as used herein includes all-E and Z-stereoisomers. Suitable derivatives are e.g. its mono-and di-esters, preferably esters of saturated alkanoic acids such as acetic, propionic, laurinic, myristinic, palmitic, stearic and succinic acid, esters of mono-unsaturated fatty acids such as oleic acid, and poly-unsaturated fatty acids such as linolic, linoleic, pentaenoic, docosahexaenoic and arachidonic acid, and mixtures thereof.

### Zeaxanthin and derivatives thereof

The term "zeaxanthin" as used herein includes all-E and Z-stereoisomers. Suitable derivatives are e.g. its mono-and di-esters, preferably esters of saturated alkanoic acids such as acetic, propionic, laurinic, myristinic, palmitic, stearic and succinic acid, esters of mono-unsaturated fatty acids such as oleic acid, and poly-unsaturated fatty acids such as linolic, linoleic, pentaenoic, docosahexaenoic and arachidonic acid, and mixtures thereof.

### (-)-Epigallocatechin gallate and derivatives thereof

The terms "(-)-epigallocatechin gallate" and "EGCG" as used herein encompass also green tea extracts containing EGCG as well as EGCG derivatives such as pharmaceutically acceptable salts.

An especially suitable EGCG is e. g. TEAVIGO^{™} (a green tea extract containing ≥ 94 % of EGCG) commercially available from DSM Nutritional Products Ltd, Kaiseraugst, Switzerland, as well as TEAVIGO^{™} TG (Tablet Grade) (a green tea extract containing ca. 88 % of EGCG admixed with ca. 3 % of pectin).

A preferred alternative for EGCG is a green tea fraction comprising at least 85.0 weight-% of EGCG and at most 2.0 weight-% of caffeine, especially a green tea fraction comprising at least 90.0 weight-% of EGCG and at most 1.6 weight-% of caffeine, whereas this green tea fraction preferably comprises at most 4.0 weight-% of epicatechin (EC), and/or at most 4.0 weight-% of catechin, and/or at most 2.0 weight-% of gallocatechin gallate (GCG), and/or at most 5.0 weight-% of epicatechin gallate (ECG).

A preferred alternative for EGCG is a green tea fraction comprising at least 91.7 weight-% of EGCG and at most 1.43 weight-% of caffeine, especially a green tea fraction comprising from 91.7 to 97.13 weight-% of EGCG, from 0 to 3.15 weight-% of epicatechin (EC), from 0 to 3.1 weight-% of catechin, from 0.2 to 1.52 weight-% of gallocatechin gallate (GCG), from 0.38 to 4.62 weight-% of epicatechin gallate (ECG) and from 0 to 1.43 weight-% of caffeine.

### Hydroxytyrosol and derivatives thereof

The term "hydroxytyrosol" as used herein relates to 'pure hydroxytyrosol' of either synthetic origin or obtainable from natural sources such as from products and by-products derived from the olive tree by extraction and/or purification. It may also be obtained together with other water-soluble polyphenols such as tyrosol and oleuropein in the form of an olive extract. Additionally the term "hydroxytyrosol" encompasses hydroxytyrosol comprising extracts obtainable e.g. from products and by-products derived from the olive tree.

Products and by-products of olive trees encompass olives, olive tree leafs, olive pulps, olive oil, olive-derived vegetation water and olive oil dregs without being limited thereto. Based on the extraction procedure the amount, respectively the ratio of the hydroxytyrosol can be easily adjusted by a person skilled in the art. Preferably, hydroxytyrosol is derived from olives that may be obtained from conventional and commercially available sources such as growers.

Examples of references that deal with the extraction hydroxytyrosol and/or oleuropein from olive leaves are WO-02/18310-A1, US-2002/0198415-A1, WO-2004/005228-A1, US-6,416,808 and US-2002/0058078-A1 which disclose a method for acidic hydrolysis of olive vegetation water for 2 to 12 months until at least 90 % of the present oleuropein has been converted. A method of extraction of phenolic compounds from olives, olive pulps, olive oil and oil mill waste water is described by Usana Inc. patents US-6,361,803 and WO-01/45514-A1 and in US-2002/0004077-A1. EP-1 582 512-A1 describes an extraction of hydroxytyrosol from olive leaves. A method for obtaining hydroxytyrosol from the vegetation water of de-pitted olives is disclosed in US-2004/0039066-A1 in paragraphs [0080]-[0091].

Preferably hydroxytyrosol is used in the form of a hydroxytyrosol containing olive extract.

Commercially available hydroxytyrosol containing olive extracts which may be used according to the invention include *e.g.* extracts from olive fruits such as Polyphen-Oil™ from Life Extension, OleaSelect™ from Indena, Hytolive^{®} from Genosa, Prolivols from Seppic, OLIVE LEAF or OLIVE Water Extract of Olea europea from Lalilab, Hitofulvic from Ebiser, hydrolysed olive leaf extract, such as described in EP-1582512-A1, olive leaf extract, rich in oleuropein, such as available from Furfural and HIDROX^{®} from CreAgri.
Preferably HIDROX^{®} from CreAgri such as HIDROX^{®} 2 % spray dried powder, HIDROX^{®} Gold freeze dried powder (9 %) and HIDROX^{®} 6 % freeze dried powder organic olive juice extract are used.

Derivatives may be esters as well as physiologically/pharmaceutically acceptable salts.

### Resveratrol

The term "resveratrol" as used herein comprises resveratrol itself and derivatives, metabolites or analogues thereof. The carbon-carbon double bond may be trans or cis and includes cis/trans mixtures. Etherified or esterified hydroxy groups may be derived from unsubstituted or substituted, straight or branched chain alkyl groups having 1 to 26 carbon atoms or from unsubstituted or substituted, straight or branched chain aliphatic, araliphatic or aromatic carboxylic acids having 1 to 26 carbon atoms. Etherified hydroxy groups may further be glycoside groups and esterified hydroxy groups may further be glucuronide or sulfate groups. Especially preferred for the purposes of the invention is (trans)-resveratrol.

### Genistein

The term "genistein" as used herein comprises the aglycone (4', 5, 7-trihydroxyisoflavone) and derivatives thereof, *e.g.*, genistein glycosides, genistein sulfates, genistein glucuronides.

According to the present invention it is advantageous to administer the active ingredients in a way that their effective daily amounts ("daily dosages") are in the ranges given below. It is thereby irrelevant if the daily dosage is applied all at once (by a single dosage) or in multiple dosages.

PUFA(s), in particular n-3 polyunsaturated fatty acids and/or its derivatives (especially triglycerides): daily dosage for humans (70 kg person): from 50 mg to 8 g, preferred daily dosage for humans (70 kg person) from 300 mg to 2000 mg.

Resveratrol: daily dosage for humans (70 kg person): 1 to 100 mg, preferred daily dosage for humans (70 kg person): 5 to 50 mg, more preferred from 20 to 30 mg.

Genistein: is daily dosage for humans (70 kg person): 1 to 150 mg, preferred daily dosage for humans (70 kg person) 20 to 60 mg, more preferred from 20 to 40 mg.

(-)-Epigallocatechin gallate: daily dosage for humans (70 kg person): 50 to 600 mg, preferred daily dosage for humans (70 kg person): 150 to 300 mg.

Hydroxytyrosol: daily dosage for humans (70 kg person) in pure form: 0.25 to 500 mg, preferred daily dosage for humans (70 kg person): 50 to 100 mg. Alternatively, olive extract according the definition above: 10 mg to 500 mg, more preferably 50 to 400 mg, and more preferred 100 to 200 mg.

β-Carotene: daily dosage for humans (70 kg person): 0.1 to 50 mg, preferred daily dosage for humans (70 kg person): 1 and 30 mg, more preferred daily dosage for humans (70 kg person): 2 to 7 mg.

Lycopene: For usual applications the daily dosage for humans (usually determined for a 70 kg person) for lycopene should not exceed 40 mg, preferably not exceed 25 mg. In some embodiments of the invention the daily dosage for humans (70 kg person) for lycopene can be between 0.1 to 40 mg, more preferably between 0.5 to 25 mg.

Lutein: For usual applications the daily dosage for humans (usually determined for a 70 kg person) for lutein not exceed 60 mg, preferably not exceed 30 mg.
In some embodiments of the invention the daily dosage for humans (70 kg person) for lutein is between 0.1 to 60 mg, more preferably between 1.0 to 30 mg.

Zeaxanthin: daily dosage for humans (70 kg person): 0.1 to 20 mg, preferred daily dosage for humans (70 kg person): 2 to 7 mg, more preferred daily dosage for humans (70 kg person): ca. 4 mg.

Biotin: daily dosage for humans (70 kg person): 10 µg to 5 mg, preferred daily dosage for humans (70 kg person): 20 µg to 2 mg, more preferred daily dosage for humans (70 kg person): 30 µg to 500 µg

Vitamin E: For humans (70 kg person) the daily dosage preferably may vary for vitamin E between 10 mg and 2 g, more preferably between 15 and 500 mg.

Vitamin C: For humans (70 kg person) the daily dosage preferably may vary for vitamin C between 50 mg and 5 g, more preferably between 200 mg and 1.5 g.

Vitamin B12: daily dosage for humans (70 kg person): 0.5 to 10 µg, preferred daily dosage for humans (70 kg person): 1 to 5 µg, more preferred daily dosage for humans (70 kg person): 2.4 to 3 µg.

Vitamin B6: daily dosage for humans (70 kg person): 0.1 to 20 mg, preferred daily dosage for humans (70 kg person): 1 to 10 mg, more preferred daily dosage for humans (70 kg person): 2.0 to 6 mg.
Vitamin B2 daily dosage for humans (70 kg person): 0.1 to 20 mg, preferred daily dosage for humans (70 kg person): 0.5 to 15 mg, more preferred daily dosage for humans (70 kg person): 1.0 to 10 mg.

Ca-D-Pantothenate daily dosage for humans (70 kg person): 0.5 to 30 mg, preferred daily dosage for humans (70 kg person): 1 to 20 mg, more preferred daily dosage for humans (70 kg person): 2.0 mg to 10 mg.

CoQ-10: daily dosage for humans (70 kg person): 1 to 100 mg, preferred daily dosage for humans (70 kg person): 5 to 60 mg.

If instead of 'pure active ingredients' an active ingredient comprising extract is used, the amount of the extract to be used may be derived from the concentration of the 'pure active ingredient' within the extract and the finding of the optimal dosage is a matter of routine experimentation for the person skilled in the art.

In all embodiments of the present invention the definitions and the preferred daily dosages for the active ingredients as described above apply.

The invention also relates to a composition for consumption by humans comprising
a) at least one component selected from the group consisting of polyunsaturated fatty acids, esters of polyunsaturated fatty acids such as ethyl esters, mono-, di- and triglyceridesters, and fish oil, in amount in the range of from 0.50 mg to 120 mg per kg bodyweight of said humans, preferably from 5 mg to 30 mg per kg bodyweight of said humans; and at least one of the components b) to m),
b) β-carotene, in an amount in the range of from 0.0014 to 0.7 mg per kg bodyweight, preferably from 0.01 and 0.5 mg per kg bodyweight of said humans;
c) lycopene, preferably in an amount in the range of from 0.0014 to 0.6 mg, more preferably between 0.007 to 0.35 mg per kg bodyweight of said humans;
d) lutein, in an amount in the range of from 0.0014 to 0.86 mg, preferably between 0.014 to 0.5 mg per kg bodyweight of said humans;
e) zeaxanthin, in an amount in the range of from 0.0014 to 0.28 mg, preferably between 0.028 to 0.1 mg per kg bodyweight of said humans;
f) vitamin E, in an amount in the range of from 0.14 mg and 30 mg, preferably between 0.2 and 7 mg per kg bodyweight of said humans;
g) vitamin C, in an amount in the range of from 0.71 mg and 70 mg, preferably between 2.5 mg and 20 mg per kg bodyweight of said humans;
h) (-)-epigallocatechin gallate, in an amount in the range of from 0.5 to 8.5 mg, preferably 2.0 to 4.3 mg per kg bodyweight of said humans; and/or
i) hydroxytyrosol, in an amount in the range of from 0.071 to 7.1 mg, preferably 0.71 to 1.42 mg per kg bodyweight of said humans;
j) genistein: in an amount in the range of from 0.014 to 2.14 mg, preferably 0.28 to 0.85 mg per kg bodyweight of said humans;
k) resveratrol: in an amount in the range of from 0.014 to 1.4 mg, preferred preferably 0.071 to 0.71 mg, more preferred from 0.28 to 0.42 mg per kg bodyweight of said humans.
l) biotin, in an amount in the range of from 0.14 µg to 71 µg, preferably 0.28 µg to 28 µg per kg bodyweight of said humans;
m) coenzyme Q 10, in an amount in the range of from 0.014 to 1.4 mg, preferably 0.071 to 0.85 mg per kg bodyweight of said humans;
wherein the amount of the component(s) b) to m) is in the range of 25 to 80 % by weight, based on the total weight of the composition.

According to the present invention the amount of the component(s) b) to m) is preferably in the range of 30 to 75 % by weight, more preferably in the range of 40 to 60 % by weight, based on the total weight of the composition.

Preferably, the invention relates to a composition for consumption by humans comprising
i) at least one component selected from the group consisting of polyunsaturated fatty acids, esters of polyunsaturated fatty acids such as ethyl esters, mono-, di- and triglyceridesters, and fish oil, in an amount in the range of from 0.50 mg to 120 mg per kg bodyweight of said humans, preferably from 5 mg to 30 mg per kg bodyweight of said humans; and
ii) at least one polyphenol selected from the group consisting of (-)-epigallocatechin gallate, genistein, resveratrol and pharmaceutically acceptable derivatives thereof, in an amount in the range of from 0.014 to 8.5 mg, preferably 0.14 to 4.3 mg per kg bodyweight of said humans; and
iii) optionally hydroxytyrosol and/or pharmaceutically acceptable derivatives thereof, in an amount in the range of from 0.014 to 7.1 mg, preferably 0.71 to 1.42 mg per kg bodyweight of said humans, and/or olive extract in an amount in the range of from 0.014 to 7.1 mg, preferably 0.71 to 1.42 mg per kg bodyweight of said humans.

In an even more preferred embodiment of the present invention this composition comprises in addition to PUFAs and/or their esters and polyphenol(s) an additional amount of lipid soluble and/or water soluble vitamins, co-enzymes, anti-oxidants, carotenoids and/or their esters with the definitions and preferences outlined above.

Furthermore, the invention relates to the use of the compositions according to the invention for supporting a healthy skin appearance and beauty, for supporting skin nourishment from inside the body via the blood stream (systemically), for fostering hydration, for moisturizing the skin, for supporting the skin barrier function, for providing protection against oxidative stress, for providing protection against UV-radiation and/or for providing a general anti-aging effect., in particular for promoting skin repair and/or regeneration upon healing of injuries and/or for promoting the physiological renewal process and thus for promoting an optimal health, a natural radiance and glow and/or a beautiful look of the skin.

In another embodiment the invention relates to a method of supporting healthy skin appearance and beauty, skin nourishment from inside the body via the blood stream (systemically), moisturizing the skin, supporting the skin barrier function, fostering hydration, providing protection against oxidative stress and/or against UV-radiation and/or providing a general anti-aging effect, in particular promoting skin repair and/or regeneration upon healing of injuries and/or promoting the physiological renewal process and thus an optimal health, a natural radiance and glow and/or a beautiful appearance of the skin comprising the step of administering a composition containing an effective amount of
i) at least one component selected from the group consisting of polyunsaturated fatty acids, esters of polyunsaturated fatty acids such as ethyl esters, mono-, di- and triglyceridesters, and fish oil; and
ii) at least one component selected from the group consisting of carotenoids, water-soluble vitamins, fat-soluble vitamins, ubichinones and polyphenols, to humans,
wherein the amount of the component(s) ii) is in the range of 25 to 80 % by weight, based on the total weight of the composition.

According to the present invention the amount of the component(s) ii) is preferably in the range of 30 to 75 % by weight, more preferably in the range of 40 to 60 % by weight, based on the total weight of the composition.

The term "an effective amount" as used herein refers to an amount necessary to obtain a physiological effect. The physiological effect may be achieved by one single dose or by repeated doses. The dosage administered may, of course, vary depending upon known factors, such as the physiological characteristics of the particular composition and its mode and route of administration; the age, health and weight of the recipient; the nature and extent of the symptoms; the kind of concurrent treatment; the frequency of treatment; and the effect desired and can be adjusted by a person skilled in the art. The necessary daily amounts may be applied in a single dosage or in multiple dosages.

The compositions according to the present invention can serve as supplements to food, feed and beverages, dietary supplements and as pharmaceutical formulations which may be solid - such as capsules or tablets - or liquid - such as solutions or suspensions. They may be administered in form of (fortified) food, dietary supplements, beverages, tablets, granules, capsules, pastes, food additives, or effervescent formulations. Further examples are cereals, cereal bars and dairy products (e. g. milk, buttermilk, soured milk, yogurt (drinks), curd, quark desserts and so on) containing the component(s) i) and ii) according to the invention.

The components according to the present invention may be administered together in one pharmaceutical form or separately in various pharmaceutical forms (such as in two different pharmaceutical forms). If two or more pharmaceutical forms are used, the pharmaceutical forms are preferably consumed by the human(s) at the same time.
"At the same time" as used herein means that the different pharmaceutical forms are orally consumed within a time period of 1 day, preferably within a time period of 1 h, more preferably within a time period of 5 minutes, even more preferably within a time period of 1 minute.

The pharmaceutical form(s) comprise the components according to the present invention and optionally a suitable excipient and/or carrier.

The term "pharmaceutical form" as used herein comprises gelcaps, capsules, powders, solid tablets (coated or non-coated), syrups, drink ampoules and sachets/pouches, preferably tablets or capsules such as hard (shell) gelatin capsules. Suitable excipient and/or carriers include maltodextrin, calcium carbonate, dicalcium phosphate, tricalcium phosphate, microcrystalline cellulose, dextrose, rice flour, magnesium stearate, stearic acid, croscarmellose sodium, sodium starch glycolate, crospovidone, sucrose, vegetable gums, lactose, methylcellulose, povidone, carboxymethylcellulose, corn starch, and the like (including mixtures thereof). Preferred carriers include calcium carbonate, magnesium stearate, maltodextrin, and mixtures thereof. The components according to the present invention are mixed with the excipient(s) and/or carrier(s) and formed into the desired form using conventional techniques. The tablet or capsule according to the present invention may be coated with an enteric coating that dissolves at a pH of about 6.0 to 7.0. A suitable enteric coating that dissolves in the small intestine but not in the stomach is cellulose acetate phthalate. Further details on techniques for formulation and administration may be found in the latest edition of Remington's Pharmaceutical Sciences (Maack Publishing Co., Easton, PA).

Thus the invention also relates to a kit for oral intake comprising
i) a pharmaceutical form (A) comprising
   a. at least one component selected from the group consisting of polyunsaturated fatty acids, esters of polyunsaturated fatty acids such as ethyl esters, mono-, di- and triglyceridesters, and fish oil; and
   b. optionally one ore more component(s) selected from the group consisting of carotenoids, water-soluble vitamins, fat-soluble vitamins, ubichinones and polyphenols and
   c. optionally a suitable excipient and/or carrier;
      and
ii) a pharmaceutical form (B) different from (A) comprising at least one component selected from the group consisting of carotenoids, water-soluble vitamins, fat-soluble vitamins, ubichinones and polyphenols with the definitions and preferences as outlined above and optionally a suitable excipient and/or carrier;
wherein form (A) and form (B) are contained separately and the separate containers are joined together in a unitary package; and
wherein the amount of one or more components chosen form the group consisting of carotenoids, water-soluble vitamins, fat-soluble vitamins, ubichinones and/or polyphenols is between 25 and 80 % by weight with regard to the daily intake of both, pharmaceutical form (A) and pharmaceutical form (B).

It is according to the present invention preferred if pharmaceutical form (A) is a capsule and pharmaceutical form (B) is a tablet.

In another preferred embodiment the present invention relates to a personal skin care kit comprising:
i) one composition for oral intake according to the invention ("oral composition") and
ii) one product suitable for topical application to the skin ("topical composition")
wherein the oral composition and the topical composition are contained separately and the separate containers are joined together in a unitary package.

According to the present invention it is preferred if the oral composition and the topical composition are applied within a time period of 1 day, preferably within a time period of 3 hours, preferably within a time period of 1 hour.

The kits of the present invention are useful for supporting a healthy skin appearance and beauty, for supporting skin nourishment from inside the body via the blood stream (systemically) and in the case of the personal care kit also from outside the body via topical application of a cosmetic composition, for fostering hydration, for moisturizing the skin, for supporting the skin barrier function, for providing protection against oxidative stress, for providing protection against UV-radiation and/or for providing a general anti-aging effect. The kits of the present invention are in particular suitable for promoting skin repair and/or regeneration upon healing of injuries and/or for promoting the physiological renewal process and thus for promoting an optimal health, a natural radiance and glow and/or a beautiful appearance of the skin.

The kits of the present invention are preferably presented to a user or potential user (hereinafter "users") in association with information which informs such users that use of the kit will provide one or more benefits, including, but not limited to, supporting a healthy skin appearance and beauty, supporting skin nourishment from inside the body via the blood stream (systemically), fostering hydration, moisturizing the skin, supporting the skin barrier function providing protection against oxidative stress, providing protection against UV-radiation and/or providing a general anti-aging effect, in particular for promoting skin repair and/or regeneration upon healing of injuries and/or for promoting the physiological renewal process and thus for promoting an optimal health, a natural radiance and glow and/or a beautiful look of the skin. Such information preferably also includes instructions for use to obtain such benefits, e.g., including a detailed description of the mode of application, especially including information about how to achieve the preferred daily dosage. By "in association with information" as used herein it is meant that the information is either directly printed on the packaging of the kit itself (including direct printing on the container per se or indirectly via a label or the like affixed to the container), or presented in a different manner including, but not limited to a brochure, print advertisement, electronic advertisement and/or other advertisement, so as to communicate the information to a consumer of the composition. Such information may accordingly comprise words, pictures, and the like.

The term "topical composition" as used herein refers to a cosmetic composition that can be topically applied to mammalian keratinous tissue.
The term "cosmetic preparation" or "cosmetic composition" as used in the present application refers to cosmetic compositions as defined under the heading "Kosmetika" in Römpp Lexikon Chemie, 10th edition 1997, Georg Thieme Verlag Stuttgart, New York.
Preferably, the topical compositions according to the present invention are in the form of a suspension or dispersion in solvents or fatty substances, or alternatively in the form of an emulsion or micro emulsion (in particular of O/W- or W/O-type), PIT-emulsion, multiple emulsion (e. g. O/W/O- or W/O/W-type), pickering emulsion, hydrogel, alcoholic gel, lipogel, one- or multiphase solution or vesicular dispersion or other usual forms, which can also be applied by pens, as masks or as sprays. If the topical composition is or comprises an emulsion it can also contain one or more anionic, nonionic, cationic or amphoteric surfactant(s).

Preferred topical compositions according to the invention are skin care preparations, decorative preparations, light protection preparations and functional preparations.

Examples of skin care preparations are, in particular, body oils, body lotions, body gels, treatment creams, skin protection ointments, shaving preparations, such as shaving foams or gels, skin powders such as baby powder, moisturizing gels, moisturizing sprays, revitalizing body sprays, cellulite gels, face and/or body moisturizers, facial and/or body cleansers, face masks, anti acne preparations and/or peeling preparations.

Examples of decorative preparations are, in particular, lipsticks, eye shadows, mascaras, dry and moist make-up formulations, rouges, powders, and/or suntan lotions.

Examples of functional preparations are cosmetic or pharmaceutical compositions containing active ingredients such as hormone preparations, vitamin preparations, vegetable extract preparations, anti-ageing preparations, and/or antimicrobial (antibacterial or antifungal) preparations without being limited thereto.

Topical compositions in accordance with the invention can be in the form of a liquid, lotion, a thickened lotion, a gel, a cream, a milk, an ointment, a paste, a powder, a make-up, or a solid tube stick and can be optionally be packaged as an aerosol and can be provided in the form of a mousse such as a aerosol mousse, a foam or a spray foam, a spray, a stick, a plaster, a cleanser, a soap or a wipe.

In accordance with the present invention, the topical composition contains at least one cosmetically active ingredient in particular for skin lightening; tanning prevention; treatment of hyperpigmentation; preventing or reducing acne, wrinkles, lines, atrophy and/or inflammation; as well as topical anesthetics; antimicrobial and/or antifungal agents; chelators and/or sequestrants; anti-cellulites agents (e.g. phytanic acid) and/or sunscreening additives and carriers and/or excipients or diluents conventionally used in topical compositions. If nothing else is stated, the excipients, additives, diluents, etc. mentioned in the following are suitable for topical compositions according to the present invention. The necessary amounts of the cosmetic and dermatological adjuvants and additives can, based on the desired product, easily be determined by the skilled person.

The cosmetically active ingredients useful herein can in some instances provide more than one benefit or operate via more than one mode of action.

Examples of cosmetically active ingredients to be used in the topical composition according to the invention comprise peptides (e.g., Matrixyl^{™} [pentapeptide derivative]), oligopeptides, wax-based synthetic peptides (e.g., octyl palmitate and tribehenin and sorbitan isostearate and palmitoyl-oligopeptide), iodopropyl butylcarbamate, glycerol, urea, guanidine (e.g. amino guanidine); vitamins and derivatives thereof such as vitamin C (ascorbic acid), vitamin A (e.g., retinoid derivatives such as retinyl palmitate or retinyl propionate), vitamin E (e.g., tocopherol acetate), vitamin B₃ (e.g. niacinamide) and vitamin B₅ (e.g. panthenol), vitamin B₆ and vitamin B₁₂, biotin, folic acid; anti-acne actives or medicaments (e.g. resorcinol, salicylic acid, and the like); antioxidants (e.g. phytosterols, lipoic acid); flavonoids (e.g. isoflavones, phytoestrogens); skin soothing and healing agents such as aloe vera extract, allantoin and the like; agents suitable for aesthetic purposes such as essential oils, fragrances, skin sensates, opacifiers, aromatic compounds (e.g., clove oil, menthol, camphor, eucalyptus oil, and eugenol), desquamatory actives, hydroxy acids such as AHA acids, poly unsaturated fatty acids, radical scavengers, farnesol, antifungal actives in particular bisabolol, alkyldiols such as 1,2-pentanediol, hexanediol or 1,2-octanediol, phytol, polyols such as phytanetriol, ceramides and pseudoceramides, amino acids, protein hydrolysates, polyunsaturated fatty acids, plant extracts like kinetin, DNA or RNA and their fragmentation products, carbohydrates, conjugated fatty acids, carnitin, carnosine, biochinonen, phytofluen, phytoen, and their corresponding derivatives, co-enzyme Q10/ubiquinone), anti-oxidants [preferably (-)-epigallocatechin galate (EGCG), hydroxytyrolsol, and/or olive extract] without being limited thereto.

Preferred examples of cosmetically active ingredients are vitamin C (ascorbic acid) and/or its derivatives (e.g. ascorbyl phosphate such as Stay C (sodium ascorbyl monophosphate) from DSM Nutritional Products Ltd.), vitamin A and/or its derivatives (e.g., retinoid derivatives such as retinyl palmitate or retinyl propionate), vitamin E and/or its derivatives (e.g., tocopherol acetate), vitamin B₆, vitamin B₁₂, biotin, co-enzyme Q10, EGCG, hydroxytyrosol and/or olive extract.

It is according to the present invention preferred if the active ingredients used in the topical composition are complementary to the active ingredients used in the oral composition with regard to the desired effects; i.e. it is preferred to use the same active ingredients in the topical and the oral composition or to use additional active ingredients in the topical composition that enhance the desired effects of the oral composition.

For example, if a sun (UV) protective effect is desired, it is advantageous to apply an oral composition according to the present invention that contains one or more active ingredients from the following table in combination with a topical composition that contains one or more active ingredients from the following table:

| **Prevention** | | **Immediate protection from acute sun damage** | |
|---|---|---|---|
| *Oral* | *Topical* | *Oral* e.g. as isotonic drink | *Topical* |
| Antioxidants Vitamins Betacarotene EGCG Resveratrol Hydroxytyrosol Coenzyme Q10 | Ascorbyl Phosphate Vitamin E Dihydroxyaceton | Folic acid Vitamin B12 Vitamins A,C, E EGCG | UV-filter substances (e.g. Parsols¹) Vitamin E |

If a sun (UV) protective effect is desired, the term "oral prevention" means intake of the active ingredient(s) months to days before sun exposure. "Topical prevention" means enrichment of the protective ingredient(s) by topical application months, days to hours before exposure to sun light.
"Immediate protection from acute sun damage" means application in minutes before and application during sun exposure. The term "protection" encompasses not only protection against sun burn (sun erythema), but also protection against damages through sunlight-induced oxidative stress and/or immune suppression and/or their consequences, i.e. photoaging.

¹The following Parsols are available from DSM Nutritional Products Ltd.:

| | |
|---|---|
| PARSOL® 1789: | 1-(4-tert-butylphenyl)-3-(4-methoxyphenyl)-propane-1,3-dione |
| PARSOL® 340: | 2-cyano-3,3-diphenyl-acrylic acid 2-ethyl-hexyl ester |
| PARSOL® 5000: | (E)-rac-1,7,7-trimethyl-3-(4-methyl-benzylidene)-bicyclo-[2.2.1]-heptan-2-one |
| PARSOL® EHS: | 2-ethylhexyl salicylate |
| PARSOL® HMS: | 3,3,5-trimethylcyclohexyl salicylate |
| PARSOL® HS: | 2-phenyl-1H-benzimidazole-5-sulphonic acid |
| PARSOL® MCX: | 3-(4-methoxy-phenyl)-propionic acid 2-ethyl-hexyl ester; 2-ethylhexyl 3-(4-methoxyphenyl)-2-propenoate |
| PARSOL® SLX: | CAS No.: 207574-74-1 |
| PARSOL® TX: | Titanium Dioxide |

| **Repair** | |
|---|---|
| *Oral* | *Topical* |
| Coenzyme Q10 | Panthenol |
| EGCG | Vitamin E |
| Biotin | Eicosapenta-5,8,11,14,17-enoic acid |
| Vitamin C | Coenzyme Q10 |
| Pantothenate | |
| Resveratrol | |
| B-Vitamins | |

The term "repair" as used in this context means application of the active ingredients after sun-light induced damage as described above has occurred.

If the prevention or treatment/reduction of cellulite is the desired effect, it is advantageous to apply an oral composition according to the present invention that contains one or more active ingredients from the following table in combination with a topical composition that contains one or more active ingredients from the following table:

| **Prevention of cellulite** | | **Treatment/reduction of cellulite** | |
|---|---|---|---|
| *Oral* | *Topical* e.g. massage cream or gel | *Oral* | *Topical* e.g. massage cream or gel |
| Betacarotene | Genistein | Betacarotene | Genistein |
| Genistein | Resveratrol | Genistein | Resveratrol |
| EGCG | Phytanic Acid | TEAVIGO^{™} | Phytanic Acid |
| Resveratrol | | Resveratrol | |

If an anti-aging effect is desired, it is advantageous to apply an oral composition according to the present invention that contains one or more active ingredients from the following table in combination with a topical composition that contains one or more active ingredients from the following table:

| **Prevention** | | **Repair** | |
|---|---|---|---|
| *Oral* | *Topical* | *Oral* | *Topical* |
| Resveratrol | Colorless | Resveratrol | Resveratrol |
| Coenzyme Q10 | carotenoids | Coenzyme Q10 | Coenzyme Q10 |
| Vitamin E | | Genistein | Vitamin E |
| Genistein | | Hydroxytyrosol | Genistein |
| Hydroxytyrosol | | Betacarotene | Milk tripeptides |
| Betacarotene | | Carotenoids | Vitamins |
| Carotenoids | | (including colorless | Panthenol |
| (including colorless | | carotenoids) | Vitamin A |
| carotenoids) | | Vitamin C | (including Retinyl |
| Vitamin C | | Vitamin A | esters) |
| Vitamin A | | | |

If the prevention or treatment/reduction of sensitive and/or dry skin is the desired effect, it is advantageous to apply an oral composition according to the present invention that contains one or more active ingredients from the following table in combination with a topical composition that contains one or more active ingredients from the following table:

| **Prevention** | | **Acute anti-inflammation, soothing** | |
|---|---|---|---|
| *Oral* | *Topical* | *Oral* | *Topical* |
| Biotin | Panthenol | Resveratrol | Resveratrol |
| Hydroxytyrosol | | Hydroxytyrosol | Hydroxytyrosol |
| Vitamin E | | | Panthenol |
| Resveratrol | | | |

The term "oral prevention" as used in this context means intake of the active ingredient(s) months to days before sensitive and/or dry skin occurs such as in winter season or after sun exposure. "Topical prevention" means enrichment of the protective ingredient(s) by topical application months, days to hours before sensitive and/or dry skin occurs.
"Acute anti-inflammation and soothing" as used in this context means application in minutes before and application when sensitive and/or dry skin is present. The term "protection" encompasses not only protection against sensitive and/or dry skin, but also normalization of sensitive and/or dry skin.

| **Repair** | |
|---|---|
| *Oral* | *Topical* |
| Biotin | Panthenol |
| Hydroxytyrosol | Vitamin E |
| Vitamins E,C | Phytanic acid |
| B-Vitamins | |

The term "repair" as used in this context means application of the active ingredients after sensitive and/or dry skin as described above has occurred.

The topical cosmetic compositions of the invention can also contain usual cosmetic adjuvants and additives, such as preservatives/ antioxidants, fatty substances/ oils, water, organic solvents, silicones, thickeners, softeners, emulsifiers, sunscreens, antifoaming agents, moisturizers, aesthetic components such as fragrances, surfactants, fillers, sequestering agents, anionic, cationic, nonionic or amphoteric polymers or mixtures thereof, propellants, acidifying or basifying agents, dyes, colorings/colorants, abrasives, absorbents, essential oils, skin sensates, astringents, antifoaming agents, pigments or nanopigments, e.g. those suited for providing a photoprotective effect by physically blocking out ultraviolet radiation, or any other ingredients usually formulated into cosmetic compositions. Such cosmetic ingredients commonly used in the skin care industry, which are suitable for use in the compositions of the present invention are e.g. described in the CTFA Cosmetic Ingredient Handbook, Second Edition (1992) without being limited thereto.

The necessary amounts of the cosmetic and dermatological adjuvants and additives can - based on the desired product - easily be chosen by a skilled person in this field and will be illustrated in the examples, without being limited hereto.

The usual cosmetic adjuvants and additives such as e.g. emulsifiers, thickeners, surface active ingredients and film formers can show synergistic effects which can be determined by the expert in the field with normal trials, or with the usual considerations regarding the formulation of cosmetic composition.

Which amount of the topical composition has to be applied, depends on the concentration of the active ingredient(s) in the product and the desired cosmetic effect(s). A typical "leave-on" composition like a skin care emulsion, for example, is usually applied in an amount of about 0.5 to about 2 mg per cm² skin. The applied amount is normally not critical, and the desired effect(s) may be achieved by using more of the composition, repeating the application of the composition and/or applying a composition which contains more of the active ingredient(s).

By "'leave-on' composition" as used herein a topical composition is meant which after having applied to the skin, is not removed intentionally. It is preferably left on the skin for a period of at least about 15 minutes, more preferably at least about 30 minutes, even more preferably at least about 1 hour, most preferably for at least several hours, e. g. up to about 12 hours.

According to the present invention, it is advantageous to (re-) apply the oral composition - and in the case of the personal skin care kit also the topical composition - on a continuous basis (repeatedly).

By "continuous (re-) application" as used herein is meant that the oral composition and - if applicable (see above) - the topical composition is/are applied at least once a day over an extended period during the subject's lifetime, preferably at least once a day for a period of about a week, more preferably at least once a day for a period of about one month, even more preferably at least once a day for about three months, even more preferably at least once a day for about six months, and most preferred at least once a day for about one year or more. Although benefits are normally obtained after shorter periods of use (e. g. after continuous application for weeks or months respectively), it,is preferred that the application continues throughout the subject's lifetime to maintain the positive effects.

The following examples are provided to further illustrate the processes and compositions of the present invention. These examples are illustrative only and are not intended to limit the scope of the invention in any way.

### Examples:

### Example 1 "Ideal skin formula I" for oral consumption

Lecithin is dissolved in the ROPUFA® 75 n-3 '75'EE (ethylester, containing min. 75% n-3 fatty acid ethyl ester, stabilized with mixed tocopherol, ascorbyl palmitate and rosemary extracts; commercially available from DSM Nutritional Products AG, Kaiseraugst, Switzerland) and the beta-Carotene 30% FS, Lutein 20% FS (extracted from Tagetes erecta in Corn Oil and stabilized with DL-alpha-Tocopherol), redivivo^{™} (lycopene) 10% FS (in cornoil, stabilized with DL-alpha-Tocopherol) (commercially available from DSM Nutritional Products AG, Kaiseraugst, Switzerland) are added, vitamin C, Hidrox®, Biotin and CoEnzyme Q10 (as ALL-Q® 10% CWS/S commercially available from DSM Nutritional Products AG, Kaiseraugst, Switzerland) are mixed in a tumbler mixer for 5 minutes. This dry powder mix is dispersed in the oily mixture of ROPUFA®, carotene and lecithin and then encapsulated in capsules according to a commonly applied procedure.

| | |
|---|---|
| Beta-carotene | 3 mg (= 10 mg beta-Carotene 30 % FS) |
| Lycopene (redivio^{™}) | 3 mg (= 30 mg redivivo^{™} (lycopene) 10 % FS) |
| Lutein | 3 mg (= 15 mg Lutein 20 % FS) |
| D/L-alpha-Tocopherol-acetate | 75 mg |
| Vitamin C | 75 mg (as fine powder) |
| ROPUFA® 75 n-3 '75'EE | 400 mg |
| Hidrox ® | 100 mg (provides 6 mg polyphenols and 2.5 mg hydroxytyrosol) |
| Biotin | 150 µg |
| CoEnzyme Q10 (ALL-Q®) | 5 mg (=50 mg ALL-Q® 10 % CWS/S) |
| Lecithin as mixed soybean phosphatides | 25 mg |

One capsule is taken per day together with a meal.

### Example 2 "Ideal skin formula II" for oral consumption

Lecithin is dissolved in the ROPUFA® 75 n-3 '75'EE (ethylester, containing min. 75% n-3 fatty acid ethyl ester, stabilized with mixed tocopherol, ascorbyl palmitate and rosemary extracts; commercially available from DSM Nutritional Products AG, Kaiseraugst, Switzerland) and the beta-Carotene 30% FS, Optisharp^{™} 20% FS (in cornoil stabilized with DL-alpha-Tocopherol) are added. TEAVIGO^{™}, vitamin C, Hidrox®, Biotin and CoEnzyme Q10 (as ALL-Q® 10% CWS/S commercially available from DSM Nutritional Products AG, Kaiseraugst, Switzerland) are mixed in a tumbler mixer for 5 minutes. This dry powder mix is dispersed in the oily mixture of ROPUFA®, carotene and lecithin and then encapsulated in capsules according to a commonly applied procedure.

| | |
|---|---|
| Beta-carotene | 6 mg (= 20 mg beta-Carotene 30 % FS) |
| Zeaxanthin | 2.0 mg (= 10 mg Optisharp^{™} 20 % FS) |
| D/L-alpha-tocopherol-acetate | 300 mg |
| Vitamin C | 100 mg |
| TEAVIGO^{™} | 150 mg |
| ROPUFA® 75 n-3 '75'EE | 400 mg |
| Hidrox ® | 100 mg (provides 6 mg polyphenols and 2.5 mg hydroxytyrosol) |
| Biotin | 300 µg |
| CoEnzyme Q10 (ALL-Q®) | 20 mg (=200 mg ALL-Q® 10 % CWS/S) |
| Lecithin as mixed soybean phosphatides | 40 mg |

One capsule is taken per day together with a meal.

### Example 3 "A kit for oral consumption"

The liquid active ingredients beta-carotene, zeaxanthin, DL-alpha-tocoherol-acetate, and ROPUFA® 75 n-3 '75'EE can be provided within a capsule whereas the solid ingredients vitamin C, Hidrox®, Biotin and CoQ10 are provided in the form of a tablet. The tablet may be prepared with commonly known tabletting excipients such as dry binders e.g. microcrystalline cellulose, lactose, other carbohydrates or carbohydrate derivatives like starch, sorbitol, mannitol dextrins etc. A disintegrant like croscarmelllose or crospovidone may be added in an appropriate amount, as well as a lubricant like mg-stearate or a similar compound, a behenate polyethyleneglycol, or any other lubricant.

### Capsule

| | |
|---|---|
| Beta-carotene | 6 mg (= 20 mg beta-Carotene 30 % FS) |
| Zeaxanthin | 2.0 mg (= 10 mg Optisharp^{™} 20 % FS) |
| D/L-alpha-tocopherol-acetate | 300 mg |
| ROPUFA® 75 n-3 '75'EE | 400 mg |
| Biotin | 300 µg |
| Lecithin as mixed soybean phosphatides | 40 mg |

This oily mix is encapsulated in capsules according to a commonly applied procedure.

### Tablet

| | |
|---|---|
| Vitamin C | 110 mg (as Ascorbic Acid 90% Granulation) |
| TEAVIGO^{™} TG | 150 mg |
| Hidrox® | 100 mg (provides 6 mg polyphenols and 2.5 mg hydroxytyrosol) |
| Biotin | 300 µg |
| CoEnzyme Q10 (ALL-Q®) | 20 mg (=200 mg ALL-Q® 10 % CWS/S) |
| Microcrystalline cellulose | 300 mg |
| Lactose | 300 mg |
| Crospovidone | 35 mg |
| Mg-Stearate | 24.7 mg |

Biotin and microcrystalline cellulose is mixed in a tumbler mixer for 10 min. Then, lactose is added and the composition mixed for 10 min again. Vitamin C, TEAVIGO^{.} TG, Hidrox®, CoQ10 and Crospovidone are combined with the other ingredients and mixed for 10 min. Finally, mg-stearate is added to the other components and mixed for another 2 min. The mixture is compressed to tablets.

In order to provide the kit for oral intake according to the invention, the tablet and the capsule are individually packed in separate containers and then packed together in a unitary form.

Per day one capsule and one tablet are taken at the same time together with a meal.

### Example 3 "A personal care kit"

A capsule as described in example 1 or 2 is used together with an anti-ageing cream. The anti-aging cream containing the ingredients indicated below can be prepared in a manner known per se.

### Anti-aging cream

| O/W emulsion | |
|---|---|
| **Ingredients** | **% (w/w)** |
| Glyceryl Myristate | 4.00 |
| Cetyl Alcohol | 2.00 |
| Steareth-2 | 2.00 |
| Steareth-21 | 2.00 |
| Isopropyl Myristate | 5.00 |
| Caprylic/Capric Triglyceride | 8.00 |
| BHT | 0.05 |
| Dimethicone | 2.00 |
| Phenoxyethanol & Methylparaben & Ethylparaben & Butylparaben & Propylparaben & Isobutylparaben | 0.80 |
| CoEnzyme Q10 (ALL-Q®) | 0.50 |
| Water | Ad 100 |
| Xanthan Gum | 0.50 |
| Disodium EDETA | 0.10 |
| Propylene Glycol | 4.00 |

In order to provide the personal care kit according to the invention, the capsule and the anti-ageing cream are individually packed in separate containers and then packed together in a unitary form.
The anti-ageing cream is applied once a day. One capsule is taken per day together with a meal.

## Claims

1. Composition for oral intake comprising
i) at least one component selected from the group consisting of polyunsaturated fatty acids, esters of polyunsaturated fatty acids - e.g. ethyl esters, mono-, di- and triglyceridesters - and fish oil; and
ii) at least one component selected from the group consisting of carotenoids, water-soluble vitamins, fat-soluble vitamins, ubichinones and polyphenols,
wherein the amount of the component(s) ii) is in the range of 25 to 80 % by weight, based on the total weight of the composition.

2. Composition according to claim 1, wherein the component i) is essentially a mixture of eicosapentaenoic acid, docosahexaenoic acid and their esters.

3. Composition according to claim 2, wherein the esters are ethyl esters.

4. Composition according to claim 1, wherein the carotenoids are selected from the group consisting of β-carotene, lutein, zeaxanthin, and lycopene and pharmaceutically acceptable derivatives thereof.

5. Composition according to claim 1, wherein the water-soluble vitamins are selected from the group consisting of the B vitamins, vitamin C, biotin and pharmaceutically acceptable salts and derivatives thereof.

6. Composition according to claim 1, wherein the fat-soluble vitamins are selected from the group consisting of vitamin A, vitamin E and pharmaceutically acceptable derivatives thereof.

7. Composition according to claim 1, wherein the ubichinones are selected from the group consisting of coenzyme Q 10, vitamin K and pharmaceutically acceptable derivatives thereof.

8. Composition according to claim 1, wherein the polyphenols are selected from the group consisting of (-)-epigallacatechin-3-gallate, hydroxytyrosol, resveratrol, genistein and pharmaceutically acceptable derivatives thereof.

9. Composition for consumption by humans comprising
i) at least one component selected from the group consisting of polyunsaturated fatty acids, esters of polyunsaturated fatty acids such as ethyl esters, mono-, di- and triglyceridesters, and fish oil; and
ii) at least one polyphenol selected from the group consisting of (-)-epigallocatechin gallate, genistein, resveratrol and pharmaceutically acceptable derivatives thereof; and
iii) optionally hydroxytyrosol, pharmaceutically acceptable derivatives thereof and/or olive extract;
wherein the amount of the component(s) ii) and iii) is in the range of 25 to 80 % by weight, based on the total weight of the composition.

10. The composition according to claim 9 further comprising lipid soluble and/or water soluble vitamins, co-enzymes, anti-oxidants, carotenoids and/or their esters.

11. Use of a composition according to any of claims 1 to 10 for supporting a healthy skin appearance and beauty, for supporting skin nourishment from inside the body via the blood stream (systemically), for fostering hydration, for moisturizing the skin, for supporting the skin barrier function, for providing protection against oxidative stress, for providing protection against UV-radiation and/or for providing a general anti-aging effect.

12. Use of a composition according to any of claims 1 to 10 for promoting skin repair and/or regeneration upon healing of injuries and/or for promoting the physiological renewal process.

13. Use of a composition according to any of claims 1 to 10 for promoting an optimal health, natural radiance and glow and/or a beautiful look of the skin.

14. Method of supporting healthy skin appearance and beauty, skin nourishment from inside the body via the blood stream (systemically), moisturizing the skin, supporting the skin barrier function, fostering hydration, providing protection against oxidative stress and/or against UV-radiation and/or providing a general anti-aging effect, in particular promoting skin repair and/or regeneration upon healing of injuries and/or promoting the physiological renewal process and thus an optimal health, a natural radiance and glow and/or a beautiful appearance of the skin comprising the step of administering a composition containing an effective amount of
i) at least one component selected from the group consisting of polyunsaturated fatty acids, esters of polyunsaturated fatty acids such as ethyl esters, mono-, di- and triglyceridesters, and fish oil; and
ii) at least one component selected from the group consisting of carotenoids, water-soluble vitamins, fat-soluble vitamins, ubichinones and polyphenols, to humans,
wherein the amount of the component(s) ii) is in the range of 25 to 80 % by weight, based on the total weight of the composition.

15. A kit for oral intake comprising
i) a pharmaceutical form (A) comprising
a. at least one component selected from the group consisting of polyunsaturated fatty acids, esters of polyunsaturated fatty acids such as ethyl esters, mono-, di- and triglyceridesters, and fish oil; and
b. optionally one ore more component(s) selected from the group consisting of carotenoids, water-soluble vitamins, fat-soluble vitamins, ubichinones and polyphenols and
c. optionally a suitable excipient and/or carrier;
and
ii) a pharmaceutical form (B) different from (A) comprising at least one component selected from the group consisting of carotenoids, water-soluble vitamins, fat-soluble vitamins, ubichinones and polyphenols with the definitions and preferences as outlined above and optionally a suitable excipient and/or carrier;
wherein form (A) and form (B) are contained separately and the separate containers are joined together in a unitary package; and
wherein the amount of one or more components chosen form the group consisting of carotenoids, water-soluble vitamins, fat-soluble vitamins, ubichinones and/or polyphenols is between 25 and 80 % by weight with regard to the daily intake of both, pharmaceutical form (A) and pharmaceutical form (B).

16. A personal care kit comprising separate containers packaged together in a unitary form comprising
i) a composition suitable for oral consumption according to any one of claims 1 to 10,
ii) a product suitable for topical application to the skin
wherein i) and ii) are contained separately and the separate containers are joined together in a unitary package.

17. A personal care kit according to claim 16, wherein the product suitable for topical application to the skin contains at least one cosmetically active ingredient selected from the group consisting of vitamin C (ascorbic acid) and/or its derivatives (e.g. ascorbyl phosphate), vitamin A and/or its derivatives (e.g. retinoid derivatives such as retinyl palmitate or retinyl propionate), vitamin E and/or its derivatives (e.g. tocopherol acetate), vitamin B₆, vitamin B₁₂, biotin, co-enzyme Q10, EGCG, hydroxytyrosol and/or olive extract..
